# FASCICULE DE BREVET EUROPEEN

(11) **EP 1 265 845 B1**
(45) Date de publication et mention de la délivrance du brevet: **08.11.2006**
(21) Numéro de dépôt: 01913956.7
(22) Date de dépôt: 07.03.2001
(51) Int. Cl.: C07C 209/48

(54) **PROCEDE D'HYDROGENATION DE FONCTIONS NITRILES EN FONCTIONS AMINES**
VERFAHREN ZUR HYDRIERUNG VON NITRILFUNKTIONEN ZU AMINFUNKTIONEN
METHOD FOR HYDROGENATING NITRILE FUNCTIONS INTO AMINE FUNCTIONS

(30) Priorité: 08.03.2000 FR 0002997
(43) Date de publication de la demande: 18.12.2002
(73) Titulaire: RHODIA POLYAMIDE INTERMEDIATES, 69192 Saint-Fons (FR)
(72) Inventeur: BOSCHAT, Vincent, F-03200 Vichy (FR); LECONTE, Philippe, F-69330 Meyzieu (FR)
(74) Mandataire: Esson, Jean-Pierre
(86) Numéro de dépôt international: PCT/FR2001/000687
(87) Numéro de publication internationale: WO 2001/066511

(56) Documents cités:
- EP-A- 0 303 550
- WO-A-95/17959
- WO-A-95/18090
- WO-A-97/10052
- WO-A-98/43941
- DE-A- 2 238 452
- FR-A- 2 068 953
- FR-A- 2 773 086

## Description

La présente invention concerne un procédé d'hydrogénation des fonctions nitriles en fonction amines.

Elle concerne plus particulièrement un procédé d'hydrogénation totale ou partielle de composés dinitriles en composés diamines ou aminonitriles.

L'hydrogénation des dinitriles en diamines correspondantes est un procédé utilisé depuis longtemps, notamment l'hydrogénation de l'adiponitrile en hexaméthylènediamine, l'une des matières de base de la préparation du polyamide 66.

Depuis quelques années est apparu un intérêt croissant à l'hydrogénation (aussi parfois appelée hémihydrogénation) des dinitriles aliphatiques en aminonitriles, notamment l'hydrogénation de l'adiponitrile en 6-aminocapronitrile conduisant soit directement, soit par l'intermédiaire du caprolactame, au polyamide 6.

Ainsi le brevet US-A-5 151 543 décrit un procédé d'hydrogénation sélective de dinitriles aliphatiques en aminonitriles correspondants, à 25-150°C et sous pression supérieure à la pression atmosphérique, en présence d'un solvant en excès molaire d'au moins 2/1 par rapport au dinitrile, le solvant contenant de l'ammoniac liquide ou un alcool de 1 à 4 atomes de carbone et une base minérale soluble dans ledit alcool, en présence d'un catalyseur Raney, l'aminonitrile obtenu étant récupéré comme produit principal.

Le brevet WO-A-93/16034 décrit un procédé de préparation de 6-aminocapronitrile par hydrogénation de l'adiponitrile, en présence d'une base minérale, d'un complexe de métal de transition de faible valence choisi parmi le chrome, le tungstène, le cobalt et le fer et de nickel de Raney comme catalyseur, sous pression d'hydrogène et à une température de 50°C à 90°C.

Le brevet WO-A-96/18603 décrit l'hémihydrogénation de dinitriles aliphatiques en aminonitriles par l'hydrogène et en présence d'un catalyseur à base de nickel ou de cobalt de Raney éventuellement dopé et d'une base minérale forte, le milieu initial d'hydrogénation comportant de l'eau, de l'aminonitrile et/ou de la diamine susceptibles de se former et du dinitrile non transformé.

Les brevets FR2773086, WO9710052, WO9517959, WO9518090 et EP0303550 décrivent un test d'hydrogénation d'adiponitrile pour apprécier l'activité et la sélectivité de différents catalyseurs consistant à ajouter le catalyseur, une base, de l'eau et un solvant dans un réacteur puis après mélange des composants d'ajouter l'adiponitrile à hydrogéner. Ces procédés ne sont pas représentatifs d'un procédé en continu comprenant une alimentation continue des différents réactifs dans un milieu réactionnel dans lesquels ils sont déjà présents.

Le brevet français 2086953 décrit un procédé continu d'hydrogénation de composés dinitriles dans lequel les différents réactifs et notamment le catalyseur et la soude sont ajoutés de manière distincte et selon des flux séparés dans le milieu réactionnel.

Tous ces procédés d'hydrogénation conduisent à l'aminonitrile recherché et sont présentés comme pouvant être mis en oeuvre en continu dans une installation industrielle.

Toutefois, la sélectivité et le rendement de ces procédés doivent être améliorés pour les rendre plus compétitifs.

Un des buts de la présente invention est de proposer un procédé continu d'hydrogénation des fonctions nitriles en présence d'un catalyseur présentant un rendement et une sélectivité améliorés.

A cet effet, l'invention propose un procédé continu d'hydrogénation de composés nitriles aliphatiques de formule (I) décrite ci-dessous en composés comprenant des fonctions amines, à l'aide en continu d'hydrogène, d'un catalyseur d'hydrogénation et d'une base minérale forte dérivant de préférence d'un métal alcalin ou alcalino-terreux.

Selon l'invention, le procédé comprend une étape de conditionnement du catalyseur consistant à mélanger le catalyseur d'hydrogénation, une quantité déterminée de base minérale forte et un solvant dans lequel la base minérale forte est peu soluble. Selon l'invention, le milieu contenant le catalyseur ainsi conditionné est alimenté dans le réacteur d'hydrogénation. Une base forte est également ajoutée dans le milieu réactionnel d'hydrogénation. La réaction d'hydrogénation est réalisée selon les conditions et procédures habituelles et déjà décrites dans la littérature.

Par catalyseur d'hydrogénation on entend, en particulier et avantageusement, les métaux de Raney comme le nickel de Raney, le cobalt de Raney, des oxydes mixtes à structure hydrotalcite, comme décrits dans le brevet WO97/10052 mais également les métaux supportés, notamment les métaux du groupe VIII de la classification périodique des éléments tels que le nickel, le cobalt, le ruthénium, le rhodium, déposés sur un support qui est généralement un oxyde métallique ou du charbon actif.
Dans le cas des métaux de Raney, leur instabilité au contact de l'air impose l'utilisation d'un milieu liquide de conservation. Ce milieu liquide est généralement de l'eau.
Selon l'invention le solvant utilisé présente une bonne affinité avec le liquide de conservation, généralement l'eau permettant ainsi d'obtenir une démixtion et formation d'une phase contenant la base minérale forte à une concentration élevée.
Dans le cas des autres catalyseurs ne requérant pas la présence d'un liquide de conservation, il peut être intéressant et avantageux d'ajouter de l'eau au mélange.
Par affinité entre le solvant et le liquide de conservation ou l'eau, il faut comprendre que ces composés sont solubles l'un dans l'autre.
Egalement, le terme peu soluble utilisé pour caractériser la solubilité de la base minérale forte dans le solvant doit être interprété comme signifiant une solubilité inférieure à 3 % en poids de la dite base dans le solvant pur.
Selon l'invention, l'ordre d'addition des composants du mélange est indifférent

Selon le procédé de l'invention, la présence du solvant provoque une démixtion de la base minérale forte ou d'une solution concentrée en base minérale forte formant une seconde phase liquide comprenant la totalité de la quantité de base ajoutée dans le mélange, cette dite phase contenant la base forte étant et restant en contact intime avec le catalyseur. De ce fait, les particules de catalyseur entrent en contact avec une solution

De ce fait, les particules de catalyseur entrent en contact avec une solution concentrée en base minérale forte permettant son conditionnement par fixation ou adsorption des molécules de base forte à la surface dudit catalyseur.

L'utilisation d'un catalyseur comprenant des molécules de base forte à sa surface permet de réaliser une hydrogénation avec un rendement et une sélectivité améliorée se traduisant notamment par une diminution des impuretés formées, comme l'illustrent les exemples donnés ci-après.

Le catalyseur d'hydrogénation peut avantageusement comporter en plus du métal catalytique, un élément dopant choisi parmi les éléments des groupes Ib, IIb, IVb, VIb, VIIb et VIII de la classification périodique des éléments telle que publiée dans Handbook of Chemistry and Physics (Weast, 5^{ème} édition de 1970-1971), et l'aluminium notamment présent dans les métaux de Raney.

Par métal de Raney, on entend notamment le nickel de Raney, le cobalt de Raney.

Les bases minérales fortes convenables pour l'invention sont les hydroxydes de métaux alcalins ou alcalino-terreux, par exemple LiOH, NaOH, KOH, RbOH, CsOH, et leurs mélanges.

Selon une autre caractéristique préférée de l'invention, le milieu liquide de conservation du métal de Raney est, de préférence, l'eau.

Selon une caractéristique de l'invention, la quantité de base forte ajoutée dans l'étape de conditionnement du catalyseur est comprise entre 0,1 mol et 50 mol par kg de catalyseur. La quantité optimale de base est déterminée pour chaque catalyseur.

Selon un mode préféré de l'invention, la base forte est ajoutée dans l'étape de conditionnement sous forme de solution concentrée ou sous forme pure.

Par ailleurs, la quantité de solvant ajouté dépend du degré de solubilité de l'eau ou du liquide de conservation dans ce solvant et du niveau de concentration désiré dans la phase contenant la base forte. Avantageusement, le rapport en poids entre le solvant et l'eau (ou liquide de conservation) sera au moins égal à 1, de préférence supérieur ou égal à 2.

Selon l'invention, le solvant est choisi parmi les composés qui ont une affinité (pouvoir de solubilisation par exemple) avec l'eau ou le liquide de conservation du métal de Raney et qui, au contraire, n'ont pas d'affinité (pouvoir de solubilisation faible) avec la base minérale forte. Par insolubilité de la base forte dans le solvant ou plus précisément dans la phase liquide formée par le solvant et l'eau ou le liquide de conservation, il faut comprendre une solubilité faible de la base, par exemple inférieure à 1 % en poids.

Dans un mode de réalisation préféré de l'invention, le solvant est avantageusement une amine, de préférence une amine correspondant à celle obtenue par la réaction d'hydrogénation ou l'ammoniac liquide dans le cas où l'hydrogénation est conduite en milieu ammoniac liquide. En effet, le choix du solvant doit permettre, avantageusement, de ne pas introduire de nouveaux produits dans le milieu d'hydrogénation et donc de permettre des procédés de séparation et éventuellement de recyclage aisés et peu coûteux donc peu pénalisant d'un point de vue technique et économique pour le procédé.

L'étape de conditionnement du catalyseur peut être réalisée sous atmosphère inerte, éventuellement sous atmosphère d'hydrogène ou sous pression d'hydrogène.

Le procédé de l'invention s'applique plus particulièrement à l'hydrogénation de dinitriles tels que l'adiponitrile en diamines telles que l'hexaméthylène diamine (HMD) ou encore à l'hydrogénation partielle ou hémihydrogénation de dinitriles comme l'adiponitrile en aminonitrile, comme l'aminocapronitrile. Cette dernière réaction est notamment intéressante pour la fabrication de lactame comme l'ε-caprolactame obtenu par hydrolyse cyclisante de l'aminonitrile.

Généralement, cette réaction d'hémihydrogénation est réalisée en présence d'eau qui représente entre 0,1 et 20% en poids du milieu réactionnel ou en présence d'un autre composé par exemple, l'ammoniac liquide, la concentration en ce composé étant avantageusement inférieure ou égale à 50% en poids du milieu réactionnel.

Ainsi, Dans un mode de réalisation particulier d'une hémihydrogénation, le milieu initial d'hydrogénation comprend de l'eau à raison d'au moins 0,5 % en poids par rapport à la totalité des composés liquides du milieu réactionnel. Le milieu comprend également une ou des diamines et/ou des aminonitriles susceptibles de se former à partir du dinitrile par hydrogénation à l'hydrogène ainsi que du dinitrile non transformé à raison pour l'ensemble de ces trois composés de 80 % à 99,5 % en poids par rapport à la totalité des composés liquides du milieu réactionnel.

Les dinitriles aliphatiques qui peuvent être mis en oeuvre dans le procédé de l'invention sont plus particulièrement les dinitriles de formule générale (I) :

NC-R-CN (I)

dans laquelle R représente un groupement alkylène ou alcénylène, linéaire ou ramifié, ayant de 1 à 12 atomes de carbone.

De préférence, on met en oeuvre dans le procédé de l'invention des dinitriles de formule (I) dans laquelle R représente un radical alkylène, linéaire ou ramifié ayant de 2 à 6 atomes de carbone.

A titre d'exemple de tels dinitriles, on peut citer notamment l'adiponitrile, le méthylglutaronitrile, l'éthylsuccinonitrile, le malononitrile, le succinonitrile, le glutaronitrile et leurs mélanges, notamment les mélanges d'adiponitrile et/ou de méthylglutaronitrile et/ou d'éthylsuccinonitrile susceptibles de provenir d'un même procédé de synthèse de l'adiponitrile.

En pratique, le cas où R = (CH₂)₄ sera le plus fréquent car cela correspond à la mise en oeuvre de l'adiponitrile (ADN) dans le présent procédé.

Dans le procédé de l'invention, on ajoute une base forte dans le milieu réactionnel d'hydrogénation identique ou différente de celle utilisée pour le conditionnement du catalyseur. Cette base forte est généralement un hydroxyde, un carbonate ou un alcanolate de métal alcalin ou de métal alcalino-terreux.

Le milieu réactionnel a une composition variant selon le type de mise en oeuvre du procédé.

En effet si le procédé est mis en oeuvre en mode discontinu comme c'est notamment le cas dans les essais à l'échelle du laboratoire ou de fabrications de faible quantité, le milieu réactionnel initial s'enrichira progressivement en aminonitrile et, en moindre proportion, en diamine, tandis que la concentration en dinitrile pourra soit - décroître si l'on charge la totalité ou la majeure partie dudit dinitrile dès le début de l'hémihydrogénation, soit demeurer relativement constante si le dinitrile est introduit progressivement au cours de la réaction.

Par contre, si le procédé est conduit en mode continu, la composition moyenne du milieu réactionnel atteint des valeurs déterminées par le taux de conversion et les sélectivités de la réaction.

L'eau est habituellement présente dans une quantité inférieure ou égale à 20 %. Préférentiellement, la teneur en eau du milieu réactionnel est comprise entre 0,5 % et 15 % en poids par rapport à l'ensemble des constituants liquides dudit milieu.

La concentration de l'aminonitrile visé et/ou de la diamine correspondante et du dinitrile non transformé dans le milieu réactionnel est généralement comprise entre 85 % et 98 % en poids par rapport à l'ensemble des liquides inclus dans ledit milieu réactionnel.

Les catalyseurs utilisés dans ce procédé d'hémihydrogénation peut être un nickel de Raney, un cobalt de Raney, comportant, outre le nickel ou le cobalt et les quantités résiduelles du métal éliminé de l'alliage d'origine lors de la préparation du catalyseur, c'est-à-dire généralement l'aluminium, un ou plusieurs autres éléments, souvent appelés dopants, tels que par exemple le chrome, le titane, le molybdène, le cuivre, le tungstène, le fer, le zinc. Parmi ces éléments dopants le chrome, le cuivre, le titane, le fer et leurs mélanges sont considérés comme les plus avantageux. Ces dopants représentent habituellement, en poids par rapport au poids de nickel ou de cobalt, de 0 % à 15 % et de préférence de 0 % à 10 %.

On peut également utiliser, avantageusement un catalyseur à base de ruthénium déposé sur un support constitué par du noir d'acétylène. Ce catalyseur peut également comprendre des éléments métalliques dopants compris dans la liste citée pour les métaux de Raney.

La quantité de catalyseur mise en oeuvre peut varier très largement en fonction notamment de la nature du catalyseur et du mode de fonctionnement adopté ou des conditions réactionnelles choisies. A titre indicatif, on peut utiliser de 0,5 % à 50 % en poids de catalyseur exprimé en poids de métal par rapport au poids total du milieu réactionnel et le plus souvent de 1 % à 35 % en poids.

Le procédé de l'invention est généralement mis en oeuvre à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 1bar (0,10 MPa) et 100 bar (10 MPa) et de préférence entre 5 bar (0,5 MPa) et 50 bar (5 MPa).

Les autres conditions qui régissent l'hydrogénation (en mode continu ou discontinu) conforme à l'invention, relèvent de dispositions techniques traditionnelles et connues en elles-mêmes.

Par ailleurs, ces conditions peuvent être modifiées pour modifier le taux de transformation du dinitrile en diamine selon qu'il est souhaité une forte sélectivité en aminonitrile ou inversement une hydrogénation totale des dinitriles en diamines.

Les exemples ci-après donnés uniquement à titre indicatif, illustrent l'invention.

Dans ces exemples les abréviations suivantes pourront être utilisées :
- ADN = adiponitrile
- ACN = aminocapronitrile
- HMD = hexaméthylène diamine
- TT = taux de transformation (% en poids d'adiponitrile transformé)
- RT = sélectivité par rapport au substrat de départ transformé (% en mole de composé ACN (RT_{ACN}) ou HMD(RT_{HMD}) obtenu par rapport à la quantité totale d'ADN transformée).

### EXEMPLE 1 - Information technique

Dans un réacteur agité on mélange 0,806g de potasse en solution dans 4,2g d'eau avec 37,8g d'hexaméthylène diamine.

Le mélange est maintenu sous agitation à 80°C. Il se forme un système biphasique. La phase organique contenant l'HMD est analysée pour déterminer la teneur en eau et en potasse. Les résultats sont les suivants :
Teneur en eau : 8,2 % en poids
Concentration en potasse : 0,0287 % en poids

La phase aqueuse est donc une solution à environ 50% en poids de potasse.

La quantité de potasse présente dans la phase organique représente 1,5% de la quantité de potasse engagée.

### Exemples 2 et 3 - Information technique

L'exemple 1 est répété mais en mélangeant 252 g de HMD, 126 g d'éthanol et 5,76 g de soude en solution dans 42 g d'eau.

L'analyse de la phase organique obtenue après agitation montre qu'elle contient 7,16 % en poids d'eau et 0,3252 % en poids de soude.

Ce résulte montre qu'environ 25% de la soude engagée se retrouve dans la phase organique qui comprend un solvant de celle-ci, à savoir l'éthanol.

Un essai analogue sans éthanol mais utilisant 378g de HMD au lieu de 252 g, permet d'obtenir une concentration en soude dans la phase organique égale à 0,0496%. Dans cet exemple 3,6% de la soude engagée se retrouve dans la phase organique.

### Exemple 4 - Information technique

De manière analogue à l'exemple 1, on mélange 20 g de Nickel de Raney contenu dans 18g d'eau avec 180,9 g d'hexaméthylène diamine et 0,896 g de potasse en solution dans 4,23 g d'eau. Le mélange est maintenu sous agitation à 80°C.

L'analyse de la phase organique à base de HMD montre qu'elle contient 10,2% en poids d'eau et 0,0123% en poids de potasse. La quantité de potasse contenue dans la phase organique représente 2,8% de la potasse engagée. 97,2 % en poids de la potasse engagée se trouve donc en contact direct avec le catalyseur.

### Exempte 5 Information technique

Dans un réacteur agité, on charge 240 g de HMD, 52 g d'eau et 6,4g de Nickel de Raney dopé avec 1,5% en poids de chrome. 0,462 ml d'une solution de potasse à 388 g/l sont ajoutés pour obtenir un rapport KOH/Ni égal à 0,5 mol/kg. Le mélange est maintenu sous agitation à une température de 50°C. le réacteur est mis sous une pression d'hydrogène de 25 bar.

40 g d'adiponitrile sont ajoutés dans le réacteur. Après 50 minutes de réaction le milieu est refroidi et analysé par chromatographie en phase gazeuse pour déterminer le taux de transformation (TT) total de l'adiponitrile (ADN), la sélectivité (RT_{ACN}) de la réaction en aminocapronitrile (ACN) et la concentration en Ipol du milieu.

Cet indice polarographique représente notamment la concentration en composés imines dans le milieu. II est déterminé par polarographie et exprimé en moles de fonction imine par tonne d'échantillon à doser.
Taux de transformation de ADN (TT) : 83,8 %
Sélectivité en ACN (RT_{ACN}) : 68,3 %
Ipol en mol/t : 21

### Exemple 6 comparatif

L'exemple 5 est répété mais avec addition de la potasse simultanément avec l'adiponitrile. Les quantités ajoutées sont identiques.

Les résultats obtenus sont les suivants :
Taux de transformation de ADN (TT) : 81,1 %
Sélectivité en ACN (RT_{ACN}): 69,7 %
Ipol en mol/t : 76

Ce résultat montre clairement l'effet de l'étape de conditionnement du catalyseur sur la pureté du produit obtenu.,

### Exemple 7 et exemple 8 comparatif

Un catalyseur à base de ruthénium dopé par 1 % poids de fer sur un support noir d'acétylène commercialisé sous la dénomination Y 70 est obtenu par le procédé suivant :

On charge 20 g de noir d'acétylène Y70 commercialisé par SN2A, dans 800 ml d'eau. La suspension est chauffée à 90°C, sous agitation. On additionne 1,8 g de Na₂CO₃, dans un total de 70 ml d'eau. Après une période de 1 heure, on additionne une solution de 2,16 g de RuCl₃ hydraté dans 120 ml d'eau. Après 1 heure, on coule une solution de 1 g de FeCl₃ hexahydraté dans un total de 70 ml d'eau. Après une nouvelle heure, on laisse refroidir le milieu jusqu'à une température de 40°C.

Après filtration, le catalyseur est lavé avec 4 fois 200 ml d'eau à 40°C.

Le catalyseur est séché en étuve durant 1 heure, à 120°C. On obtient 21,3 g de catalyseur.

Avant test, il est séché en étuve durant 10 heures, à 80°C, sous pression réduite. Dans 36 g de HMD, on ajoute 2,4 g de catalyseur préparé selon le procédé ci-dessus, 4,8g d'eau et 5 g de potasse 15N.

Le milieu est mélangé à une température de 80°C et mis sous une pression d'hydrogène de 2,5 Mpa. Dans ce milieu 36 g d'adiponitrile sont ajoutés.

Après réaction, le milieu est analysé.

On obtient les résultats suivants :
- temps de réaction: 105 min
- TT de l'ADN : 67 %
- RT_{ACN} 75 %
- Ipoi 35 mole/t

Un essai utilisant le mode opératoire de l'exemple comparatif 6 et les masses et produits engagés dans l'exemple 7 ci-dessus, notamment le même catalyseur a donné les résultats suivants :
- temps de réaction: 110 min
- TT de l'ADN : 68,5 %
- RT_{ACN} 73 %
- Ipol 92 mole/t

## Revendications

1. Procédé continu d'hydrogénation de composés nitriles aliphatiques de formule générale I suivante :
NC-R-CN (I)
dans laquelle R représente un groupement alkylène ou alcénylène, linéaire ou ramifié, ayant de 1 à 12 atomes de carbone en composés comprenant des fonctions amines, consistant à alimenter, en continu dans un réacteur, de l'hydrogène, un catalyseur d'hydrogénation, une base minérale forte dérivant d'un métal alcalin ou alcalino-terreux et le composé nitrile à hydrogéner, **caractérisé en ce qu'**il comprend une étape de conditionnement du catalyseur consistant à mélanger, préalablement à son alimentation dans le milieu réactionnel d'hydrogénation,le catalyseur avec une quantité déterminée de base minérale forte à associer au catalyseur comprise entre 0,1 mol et 50 mol par kg de catalyseur et un solvant dans lequel la base minérale forte a une solubilité inférieure à 3 % en poids dans le solvant pur, ledit mélange contenant le catalyseur conditionné étant alimenté en continu dans le milieu réactionnel d'hydrogénation comprenant le composé à hydrogéner et éventuellement un solvant, et **en ce qu'**une base forte est ajoutée dans le milieu réactionnel d'hydrogénation.

2. Procédé selon la revendication 1, **caractérisé en ce que** le catalyseur d'hydrogénation est choisi dans le groupe comprenant les métaux de Raney, les métaux du groupe VIII de la classification périodique des éléments déposés sur un support, les oxydes mixtes à structure hydrotalcite.

3. Procédé selon la revendication 2, **caractérisé en ce que** le métal de Raney est le nickel de Raney, le cobalt de Raney.

4. Procédé selon la revendication 2, **caractérisé en ce que** les métaux du groupe VIII sont choisis dans le groupe comprenant le nickel, le cobalt, le ruthénium, le rhodium, le support étant choisi parmi les oxydes métalliques, le charbon actif, les noirs d'acétylène.

5. Procédé selon l'une des revendications précédentes, **caractérisé en ce que** quand un liquide appelé de conservation est associé avec la mise en oeuvre du catalyseur notamment un catalyseur à base de métal de Raney, le solvant précité et ledit liquide de conservation sont solubles l'un dans l'autre, la base minérale forte étant peu soluble dans la solution du solvant et du liquide de conservation.

6. Procédé selon l'une des revendications précédentes, **caractérisé en ce que** le mélange de conditionnement du catalyseur comprend de l'eau.

7. Procédé selon la revendication 5 ou 6, **caractérisé en ce que** l'eau est un liquide de conservation du catalyseur d'hydrogénation.

8. Procédé selon l'une des revendications précédentes, **caractérisé en ce que** le solvant est un composé comprenant une ou plusieurs fonctions amines ou de l'ammoniac liquide.

9. Procédé selon la revendication 8, **caractérisé en ce que** le solvant est un composé aminé formé par la réaction d'hydrogénation ou identique à un composé formé par la réaction d'hydrogénation.

10. Procédé selon l'une des revendications précédentes, **caractérisé en ce que** la base minérale forte est ajoutée dans le milieu liquide de conservation du métal de Raney avant l'addition du solvant.

11. Procédé selon l'une des revendications précédentes, **caractérisé en ce que** l'étape de conditionnement du catalyseur est réalisée sous atmosphère inerte.

12. Procédé selon l'une des revendications précédentes, **caractérisé en ce que** l'étape de conditionnement du catalyseur est réalisée sous atmosphère ou pression d'hydrogène.

13. océdé selon l'une des revendications précédentes, **caractérisé en ce qu'**il consiste à hydrogéner le composé dinitrile en aminoalkylènitrile et/ou en diamines.

14. océdé selon la revendication 13, **caractérisé en ce que** le dinitrile est l'adiponitrile, et **en ce qu'**il consiste à hydrogéner l'adiponitrile en aminocapronitrile et/ou hexaméthylène diamine.

15. rocédé selon l'une des revendications 13 ou 14, **caractérisé en ce que** le solvant est l'hexaméthylène diamine.

16. océdé selon l'une des revendications précédentes, **caractérisé en ce que** le rapport massique entre le solvant et le milieu liquide de conservation du catalyseur ou l'eau dans le mélange de conditionnement du catalyseur est au moins égal à 1.

17. rocédé selon l'une des revendications précédentes, **caractérisé en ce que** la concentration en catalyseur dans le mélange de conditionnement est inférieure ou égal à 30 % en poids, exprimé en poids de métal.

18. océdé selon l'une des revendications précédentes, **caractérisé en ce que** le catalyseur comprend des éléments dopants.

19. océdé selon la revendication 18, **caractérisé en ce que** le métal de Raney est le nickel de Raney, le ou les éléments dopants étant choisi parmi les éléments du groupe IB, IIb, IVb, VIIb et VIII de la classification périodique des éléments.

20. océdé selon la revendication 18, **caractérisé en ce que** le métal de Raney est le cobalt de Raney, le ou les éléments dopants, étant choisis dans le groupe comprenant les éléments des groupes Ib, IIb, IVb, VIb, VIIb et VIII de la classification périodique des éléments.

## Claims

1. Continuous process for the hydrogenation of aliphatic nitrile compounds of following general formula I:
NC-R-CN (I)
in which R represents a linear or branched alkylene or alkenylene group having from 1 to 12 carbon atoms, to compounds comprising amine functional groups, which consists in feeding, continuously into a reactor, hydrogen, a hydrogenation catalyst, a strong inorganic base deriving from an alkali metal or alkaline earth metal and the nitrile compound to be hydrogenated, **characterized in that** it comprises a stage of conditioning the catalyst which consists in mixing the catalyst, prior to feeding it into the hydrogenation reaction medium, with a predetermined amount of strong inorganic base to be associated with the catalyst of between 0.1 mol and 50 mol per kg of catalyst and a solvent in which the strong inorganic base has a solubility of less than 3% by weight in the pure solvent, the said mixture comprising the conditioned catalyst being fed continuously into the hydrogenation reaction medium comprising the compound to be hydrogenated and optionally a solvent, and **in that** a strong base is added to the hydrogenation reaction medium.

2. Process according to Claim 1, **characterized in that** the hydrogenation catalyst is chosen from the group consisting of Raney metals, metals from Group VIII of the Periodic Table of the Elements deposited on a support, and mixed oxides with a hydrotalcite structure.

3. Process according to Claim 2, **characterized in that** the Raney metal is Raney nickel or Raney cobalt.

4. Process according to Claim 2, **characterized in that** the metals from Group VIII are chosen from the group consisting of nickel, cobalt, ruthenium and rhodium, the support being chosen from metal oxides, active charcoal or acetylene blacks.

5. Process according to one of the preceding claims, **characterized in that**, when a liquid known as a storage liquid is associated with the use of the catalyst, in particular a catalyst based on Raney metal, the abovementioned solvent and the said storage liquid are soluble in one another, the strong inorganic base not being very soluble in the solution of the solvent and of the storage liquid.

6. Process according to one of the preceding claims, **characterized in that** the mixture for conditioning the catalyst comprises water.

7. Process according to Claim 5 or 6, **characterized in that** water is a liquid for storage of the hydrogenation catalyst.

8. Process according to one of the preceding claims, **characterized in that** the solvent is a compound comprising one or more amine functional groups or liquid ammonia.

9. Process according to Claim 8, **characterized in that** the solvent is an amine compound formed by the hydrogenation reaction or is identical to a compound formed by the hydrogenation reaction.

10. Process according to one of the preceding claims, **characterized in that** the strong inorganic base is added to the liquid medium for storage of the Raney metal before the addition of the solvent.

11. Process according to one of the preceding claims, **characterized in that** the stage of conditioning the catalyst is carried out under an inert atmosphere.

12. Process according to one of the preceding claims, **characterized in that** the stage of conditioning the catalyst is carried out under a hydrogen atmosphere or hydrogen pressure.

13. Process according to one of the preceding claims, **characterized in that** it consists in hydrogenating the dinitrile compound to aminoalkylnitrile and/or to diamine.

14. Process according to Claim 13, **characterized in that** the dinitrile is adiponitrile and **in that** it consists in hydrogenating adiponitrile to aminocapronitrile and/or hexamethylenediamine.

15. Process according to either of Claims 13 and 14, **characterized in that** the solvent is hexamethylenediamine.

16. Process according to one of the preceding claims, **characterized in that** the ratio by mass of the solvent to the liquid medium for storage of the catalyst or the water in the mixture for conditioning the catalyst is at least equal to 1.

17. Process according to one of the preceding claims, **characterized in that** the concentration of catalyst in the conditioning mixture is less than or equal to 30% by weight, expressed as weight of metal.

18. Process according to one of the preceding claims, **characterized in that** the catalyst comprises doping elements.

19. Process according to Claim 18, **characterized in that** the Raney metal is Raney nickel, the doping element or elements being chosen from the elements from Groups Ib, IIb, IVb, VIIB and VIII of the Periodic Table of the Elements.

20. Process according to Claim 18, **characterized in that** the Raney metal is Raney cobalt, the doping element or elements being chosen from the group consisting of the elements from Groups Ib, IIb, IVb, VIb, VIIB and VIII of the Periodic Table of the Elements.

## Patentansprüche

1. Kontinuierliches Verfahren zur Hydrierung von aliphatischen Nitrilverbindungen der folgenden allgemeinen Formel (I)
NC-R-CN (I)
in der
R eine lineare oder verzweigte Gruppe Alkylen oder Alkenylen mit 1 bis 12 Kohlenstoffatomen darstellt, zu Verbindungen, die Aminfunktionen umfassen,
das darin besteht, kontinuierlich in einen Reaktor Wasserstoff, einen Hydrierungskatalysator, eine starke Mineralbase, abgeleitet von einem Alkalimetall oder Erdalkalimetall, und die zu hydrierende Nitrilverbindung einzuspeisen, **dadurch gekennzeichnet, daß** es eine Stufe der Konditionierung des Katalysators umfaßt, die darin besteht, vor seinem Einspeisen in das Reaktionsmedium der Hydrierung den Katalysator mit einer bestimmten Menge von starker Mineralbase zu vermischen und an den Katalysator zu assoziieren, und zwar zwischen einschließlich 0,1 mol und 50 mol/kg Katalysator, und mit einem Lösungsmittel zu vermischen, in dem die starke Mineralbase eine Löslichkeit von unter 3 Gew.-% in dem reinen Lösungsmittel besitzt, wobei die genannte Mischung, die den konditionierten Katalysator enthält, kontinuierlich in das Reaktionsmedium der Hydrierung eingespeist wird, das die zu hydrierende Verbindung und gegebenenfalls ein Lösungsmittel umfaßt, und **dadurch**, daß eine starke Base zu dem Reaktionsmedium der Hydrierung gegeben wird.

2. Verfahren nach Anspruch 1, **dadurch gekennzeichnet, daß** der Hydrierungskatalysator aus der Gruppe gewählt wird, welche die Metalle von Raney, die Metalle der Gruppe VIII des Periodensystems der Elemente, aufgebracht auf einen Träger, und die gemischten Oxide mit Hydrotalcit-Struktur umfaßt.

3. Verfahren nach Anspruch 2, **dadurch gekennzeichnet, daß** das Raney-Metall Raney-Nickel, Raney-Cobalt ist.

4. Verfahren nach Anspruch 2, **dadurch gekennzeichnet, daß** die Metalle der Gruppe VIII aus der Gruppe gewählt werden, die Nickel, Cobalt, Ruthenium, Rhodium umfaßt und der Träger unter den Metalloxiden, Aktivkohle und Acetylenruß ausgewählt wird.

5. Verfahren nach einem der vorstehenden Ansprüche, **dadurch gekennzeichnet, daß** in dem Fall, wo eine sogenannte Konservierungsflüssigkeit mit dem Einsatz des Katalysators, insbesondere einem Katalysator auf der Basis eines Raney-Metalls, assoziiert wird, das genannte Lösungsmittel und die genannte Konservierungsflüssigkeit untereinander löslich sind, wobei die starke Mineralbase in der Lösung des Lösungsmittels und der Konservierungsflüssigkeit wenig löslich ist.

6. Verfahren nach einem der vorstehenden Ansprüche, **dadurch gekennzeichnet, daß** die Mischung zur Konditionierung des Katalysators Wasser enthält.

7. Verfahren nach Anspruch 5 oder 6, **dadurch gekennzeichnet, daß** das Wasser eine Konservierungsflüssigkeit des Hydrierungskatalysators ist.

8. Verfahren nach einem der vorstehenden Ansprüche, **dadurch gekennzeichnet, daß** das Lösungsmittel eine Verbindung ist, die eine oder mehrere Aminfunktionen oder flüssigen Ammoniak umfaßt.

9. Verfahren nach Anspruch 8, **dadurch gekennzeichnet, daß** das Lösungsmittel eine Aminverbindung ist, gebildet durch die Reaktion der Hydrierung, oder identisch mit einer Verbindung ist, die durch die Reaktion der Hydrierung gebildet wird.

10. Verfahren nach einem der vorstehenden Ansprüche, **dadurch gekennzeichnet, daß** die starke Mineralbase in das Medium der Konservierungsflüssigkeit des Raney-Metalls vor dem Zusetzen des Lösungsmittels gegeben wird.

11. Verfahren nach einem der vorstehenden Ansprüche, **dadurch gekennzeichnet, daß** die Stufe der Konditionierung des Katalysators unter inerter Atmosphäre realisiert wird.

12. Verfahren nach einem der vorstehenden Ansprüche, **dadurch gekennzeichnet, daß** die Stufe der Konditionierung des Katalysators unter Atmosphäre oder unter Druck von Wasserstoff realisiert wird.

13. Verfahren nach einem der vorstehenden Ansprüche, **dadurch gekennzeichnet, daß** es darin besteht, die Dinitril-Verbindung zu Aminoalkylnitril und/oder Diaminen zu hydrieren.

14. Verfahren nach Anspruch 13, **dadurch gekennzeichnet, daß** das Dinitril Adiponitril ist, und **dadurch**, daß es darin besteht, Adiponitril zu Aminocapronitril und/oder Hexamethylendiamin zu hydrieren.

15. Verfahren nach einem der Ansprüche 13 oder 14, **dadurch gekennzeichnet, daß** das Lösungsmittel Hexamethylendiamin ist.

16. Verfahren nach einem der vorstehenden Ansprüche, **dadurch gekennzeichnet, daß** das Masseverhältnis zwischen dem Lösungsmittel und dem Medium der Konservierungsflüssigkeit des Katalysators oder dem Wasser in der Mischung zur Konditionierung des Katalysators mindestens gleich 1 ist.

17. Verfahren nach einem der vorstehenden Ansprüche, **dadurch gekennzeichnet, daß** die Konzentration an Katalysator in der Mischung zur Konditionierung unter oder gleich 30 Gew.-% beträgt, ausgedrückt in Gewicht Metall.

18. Verfahren nach einem der vorstehenden Ansprüche, **dadurch gekennzeichnet, daß** der Katalysator Dotierungselemente umfaßt.

19. Verfahren nach Anspruch 18, **dadurch gekennzeichnet, daß** das Raney-Metall Raney-Nickel ist und das oder die Dotierungselemente unter den Elementen der Gruppe IB, IIb, IVb, VIIb und VIII des Periodensystems der Elemente ausgewählt werden.

20. Verfahren nach Anspruch 18, **dadurch gekennzeichnet, daß** das Raney-Metall Raney-Cobalt ist und das oder die Dotierungselemente aus der Gruppe gewählt werden, welche die Elemente der Gruppen Ib, IIb, IVb, VIb, VIIb und VIII des Periodensystems der Elemente umfaßt.
